# EUROPEAN PATENT SPECIFICATION

(11) **EP 4 482 881 B1**
(45) Date of publication and mention of the grant of the patent: **12.08.2026**
(21) Application number: 23704146.2
(22) Date of filing: 13.02.2023
(51) Int. Cl.: C08F 2/50, C07C 67/03, C09D 11/101, C09D 11/107, C09D 167/07, C09D 175/16

(54) **PHOTOINITIATORS**
FOTOINITIATOREN
PHOTO-INITIATEURS

(30) Priority: 24.02.2022 IT 202200003473
(43) Date of publication of application: 01.01.2025
(73) Proprietor: IGM Resins Italia S.r.l., 20154 Milano (IT)
(72) Inventor: MORONE, Marika, 22030 Lipomo (CO) (IT); RAZZANO, Vincenzo, 20060 Bussero (MI) (IT) (IT)
(74) Representative: Gislon, Gabriele
(86) International application number: PCT/EP2023/053442
(87) International publication number: WO 2023/161049

(56) References cited:
- EP-A1- 2 617 705
- WO-A1-2010/081749
- WO-A1-2015/010729

## Description

### PRIOR ART

In the last years, the design and the development of new photoinitiators (PIs) has been attracted increasing attention due to the large amount of photoinitiators that have been or are going to be banned as toxic or reprotoxic.

Various attempts were made to develop new photoinitiators able to mimic standard photoinitiators or to overcome problems such as yellowing, higher line speed, curing under LED lamps.

Unfortunately, when used in standard applications, also these photoinitiators show some limitations, such as low surface curing and, most importantly, post-cure yellowing, which makes said PI unsuitable in polymerizable systems involving transparent varnishes.

WO2015/010729 discloses certain polyols bearing 4-phenylbenzophenone residues, which are said to present one or more advantages among which reduced migration, odour and high reactivity.

Unfortunately, these photoinitiators still show an high yellowing after curing and a low reactivity under LED lamps.

There is therefore a constant need of new technical solutions able to provide good performances during polymerization while allowing to limit post-cure yellowing.

### AIMS OF THE INVENTION

It is a first aim of the invention to provide for novel photoinitiators (PIs), their use as photoinitiators and photocurable compositions comprising them.

It is a further aim of the invention to provide for photocurable compositions comprising the novel PIs of the invention.

Is a further aim of the invention to provide for processes for photocuring ethylenically unsaturated compounds using the novel PIs of the invention, as well as articles of manufacture made by said process.

### DESCRIPTION OF THE INVENTION

It was now surprisingly found that certain photoinitiatiors well react to UVA, UVB and UVC wavelength ranges as well as, and more preferably react to LED sources emitting in the range from 350 to 420 nm, while maintaining a low post cure yellowing which represents a technical progress compared to the prior art.

Indeed, it is well known that good performances under LED lamp are always coupled to a high post-cure yellowing, which is highly undesirable. According to one of its aspects, the present invention relates to a compound of formula (I) 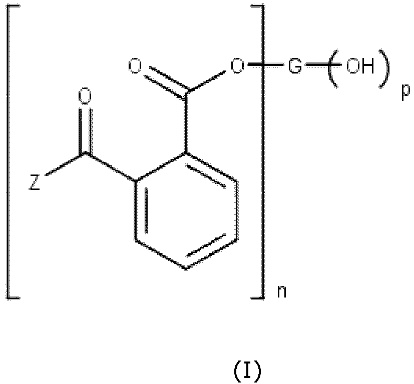 wherein
- G: is the residue of optionally ethoxylated and/or propoxylated monomeric, oligomeric or polymeric polyols G-(OH)ₙ₊ₚ;
- n: is from 1 to 8;
- p: is from 0 to 7;
- n+p: is from 2 to 8;
- and Z: is selected from (i), (ii) and (iii) 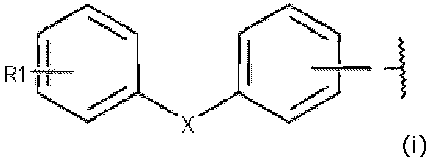 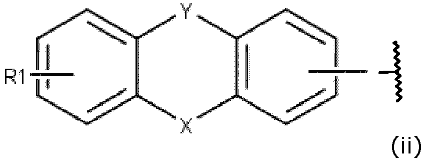 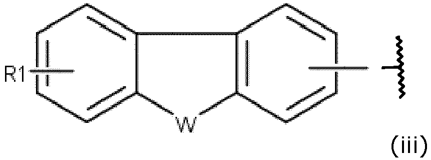
wherein
- X, Y and W: each independently, are selected from O, S and C(R2)(R3);
- R1: is selected from hydrogen, halogen, substituted or unsubstituted C1-C20 alkyl, C5-C7 cycloalkyl, C3-C12 alkenyl, -O(C3-C12 alkenyl), -S(C3-C12 alkenyl), aryl, substituted or unsubstituted C1-C20 alkoxy, C1-C50 alkyl which is interrupted by one or more oxygens, C5-C7 cycloalkoxy, aryloxy, substituted or unsubstituted C1-C20 alkylthio, arylthio, substituted or unsubstituted (C1-C12alkyl)₂amino, (C5-C7cycloalkyl)₂amino, morpholino, piperidino, piperazino and N(C1-C12alkyl)piperazino;
- R2 and R3: each independently, are selected from H, C1-C12 alkyl, OH and C1-C10 alkoxy;
and the wavy line shows the bonds linking to the keto groups of formula (I).

According to an alternative embodiment, X, Y and W are selected from O, S, C(R2)(R3) and N(R4) wherein R4 is selected from hydrogen, substituted or unsubstituted aryl and C1-C8 alkyl.

According to the invention, G-(OH)ₙ₊ₚ is selected from a monomeric, an oligomeric, a polymeric polyol and mixtures thereof, which can be optionally ethoxylated and/or propoxylated.

Examples of suitable monomeric and oligomeric polyols are ethylene glycol, propylene glycol, 1,2-butandiol, 1,2-propandiol. 1,2-hexandiol, diethanolamine, N-methyldiethanolamine, glycerol, di-glycerol, triglycerol, triethanolamine, trimethylol propane, di-trimethylol propane, pentaerythritol, di-pentaeritrithol, sugar alcohols, such as sorbitol, mannitol, xylitol and mixtures thereof.

Examples of polymeric polyols are alkoxylated compounds, polyhydroxy polyethers, which can be both aliphatic or aromatic, polyhydroxy polyesters, polyhydroxy polyamides, polyhydroxy polyimides, polyhydroxy polycarbonates and styrene-allyl alcohols copolymers.

The alkoxylated polyols are particularly preferred for the realization of the present invention. Examples of such alkoxylated polyols are monomeric and oligomeric polyols mentioned above, which have been alkoxylated, for example ethoxylated and/or propoxylated and/or butoxylated.

Other suitable examples are linear or branched polyamines, which have been alkoxylated, and polyalkoxylated diamines, such as ethoxylated ethylene diamine and ethoxylated 1,3-propylene diamine.

In the alkoxylated compounds of the invention, each group reactive toward the alkylene oxide can bring from 0 to 15 alkoxy units, preferably from 1 to 6 alkoxy units.

In a preferred embodiment G-(OH)ₙ₊ₚ is selected from monomeric and oligomeric polyols.

In another preferred embodiment G-(OH)ₙ₊ₚ is selected from monomeric and oligomeric polyols which have been ethoxylated and/or propoxylated. Preferably, G-(OH)ₙ₊ₚ has a number average molecular weight not greater than 1,500 Da, more preferably not greater than 1,000 Da and most preferably not greater than 800 Da.

Preferably, G-(OH)ₙ₊ₚ is selected from glycerol, ethoxylated and/or propoxylated glycerol, di-glycerol, ethoxylated and/or propoxylated di-glycerol, trimethylolpropane, ethoxylated and/or propoxylated trimethylolpropane, di-trimethylolpropane, ethoxylated and/or propoxylated di-trimethylolpropane, penthaerythritol, ethoxylated and/or propoxylated penthaerythritol, di-penthaerythritol, ethoxylated and/or propoxylated di-penthaerythritol, sorbitol, ethoxylated and/or propoxylated sorbitol, triethanolamine, and ethoxylated and/or propoxylated triethanolamine.

Examples of G-(OH)ₙ₊ₚ are the following: 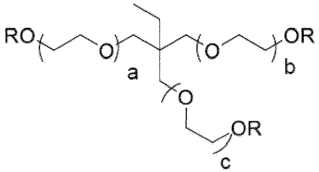
presently marketed under the trademark Polyhol 3380 (from PERSTORP); 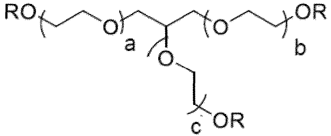
presently marketed under the trademark AIONICO GL/609 (from Lamberti SpA) or under the trademark Polyglycol G 500 (from Clariant);
wherein (a), (b) and (c) represent the number of ethoxy units which are,
each independently, from 0 to 15, preferably from 1 to 6; and

PEG having weight average molecular weight of about 200 kDa.

Preferably, n+p is from 2 to 8, and more preferably from 3 to 6.

Preferably, n is from 1 to 6 and preferably from 2 to 6, more preferably 3 to 6, most preferably 3 to 5.

Preferably, p is from 0 to 6 and more preferably from 0 to 3.

It is evident that when p is different from 0, compounds of Formula (I) have free alcoholic groups.

Preferably, R1 is hydrogen, halogen, C1-C20 alkyl, C1-C20 alkoxy, C1-C20 thioalkyl, substituted or unsubstituted (C1-C12alkyl)₂amino, morpholino, piperidino, piperazino and N(C1-C12alkyl)piperazino; more preferably R1 is hydrogen or C1-C20 alkyl, most preferably hydrogen.

Preferably, R2 and R3 are both hydrogen.

According to preferred embodiment Z is (i).

According to preferred embodiment X is S, O, preferably S.

According to preferred embodiment Z is (i) and X is S, O, preferably S.

According to preferred embodiment Z is (iii).

According to preferred embodiment W is C(R2)(R3).

According to preferred embodiment Z is (iii) and W is C(R2)(R3).

According to preferred embodiment Z is (iii), W is C(R2)(R3) and R2 and R3 are both hydrogen.

When X, Y or W are S, oxidized forms of sulfur to sulfone or sulfoxide can also exist and are also encompassed within the scope of the protection of the present invention.

In the present description the expressions "alkyl" or "alkyl group" mean, where not differently indicated, a linear or branched, saturated alkyl chain containing the given number of carbon atoms and includes all possible isomer for each number of carbon atoms in the alkyl group, i.e. for three carbon atoms: n-propyl and isopropyl; for four carbon atoms: n-butyl, isobutyl and tertiary-butyl; for five carbon atoms: n-pentyl, 1,1-dimethylpropyl, 2,2-dimethylpropyl and 2-methyl-butyl, etc..

"Alkenyl" or "alkenyl group" mean an unsaturated group containing from 2 to 12 carbon atoms, preferably C3 to C12 carbon atoms, which can be, for example, allyl, methallyl or undecenyl.

The expressions "cycloalkyl" or "cycloalkyl group" mean, where not differently indicated, an aliphatic ring preferably containing 5 or 6 carbon atoms which can be cyclopentyl or cyclohexyl.

The expressions "aryl" or "aryl group" include, but are not limited to, a substituted or unsubstituted phenyl group, a substituted or unsubstituted naphthyl group, an anthracenyl group, an indenyl group, a fluorenyl group, preferably a substituted or unsubstituted phenyl group or a substituted or unsubstituted naphthyl group.

The expression "C1-C50 alkyl which is interrupted by one or more oxygens" means that, in case more than one oxygen atom is present, said oxygen atoms are separated from one another by at least one methylene group, i.e. the oxygen atoms are non-consecutive. Preferably, oxygen atoms are separated by an ethylene or n-propylene chain. Preferably 1 to 20 oxygen atoms are present, more preferably 2 to 18. Examples include, but are not limited to, the following: -O-CH₂-OCH₃, -O-CH₂CH₂-OCH₂CH₃, -O-[CH₂CH₂O]ᵥCH₃, -O-[CH₂CH₂O]ᵥOH, -O-[CH₂CH₂O]ᵥCH₂CH₃, -CH₂-O-[CH₂CH₂O]ᵥCH₃ with v=1-24, -O-[CH₂CH₂CH₂O]ₚOH, -O-[CH₂CH₂CH₂O]ₚCH₃, -O-[CH₂CH₂CH₂O]ₚCH₂CH₃, -CH₂-O-[CH₂CH₂CH₂O]ₚCH₃ with p=1-16.

When a group is defined as possibly "substituted", if not otherwise specifically defined, the term "substituted" herein means that said group bears one or more substituents, said substituents being preferably selected from halogen atoms, alkyl, aryl, cycloalkyl, alkoxy, aryloxy, alkylamino, dialkylamino, alkylthio or arylthio, heterocyclic and phosphorous or silicon containing groups. More preferably the term "substituted" herein indicates one or more groups selected from methyl, ethyl, isopropyl, tert-butyl, phenyl, trifluoromethyl, cyano, acetyl, ethoxycarbonyl, carboxyl, carboxylate, amino, methylamino, dimethylamino, ethylamino, diethylamino, isopropylamino, diisopropylamino, cyclohexylamino, dicyclohexylamino, acetylamino, piperidino, pyrrolidyl, methoxy, ethoxy, propoxy, isopropoxy, butoxy, pentyloxy, phenoxy, hydroxyl, acetoxy, - PO₃H, methylthio, ethylthio, i-propylthio, n-propylthio, phenyltio, mercapto, acetylthio, thiocyano, methylsulfinyl, methylsulfonyl, dimethylsulfonyl, sulfonate groups, fluorine atom, chlorine atom, bromine atom, iodine atom, trimethylsilyl, pentamethyldisilyl, triethylsilyl, trimethylstannyl, furyl, thienyl, pyridyl and morpholino. Among said substituents, electron donating groups such as alkoxy groups, for example methoxy, ethoxy, isopropoxy, tert-butoxy or phenoxy groups, methyl, ethyl, isopropyl, hydroxyl, acetoxy, benzoyloxy groups, etc. or a thioalkyl group, such as methylthio, ethylthio, n-propylthio, i-propylthio, butylthio, pentylthio, or a arylthio group, such as phenylthio, are preferred.

The compound of the invention can be prepared according to any suitable process. For example, it can be prepared by reacting a compound Z-H, wherein Z is as above defined, with phthalic anhydride in a suitable organic solvent and in the presence of a Lewis catalyst to give the compound of formula (II); then reacting said compound (II) with the desired polyol in an organic suitable solvent in the presence of p-toluensulfonic acid, to provide the compound of formula (I).

Suitable organic solvents for the preparation of the compound of formula (II) are, for instance, haloaromatic solvents, such as chlorobenzene. Suitable organic solvents for the preparation of the compound of formula (I) are, for instance, aromatic solvents, such as toluene, xylene and the like.

The skilled in the art is perfectly able to perform the above chemical reactions.

Details of the process of the invention are anyway reported in the Experimental Section of the present description.

Particularly preferred PIs according to the invention are the following

| | |
|---|---|
| | 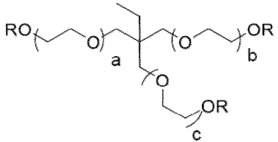 |
| R = | 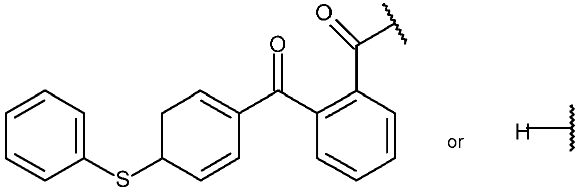 |
| | 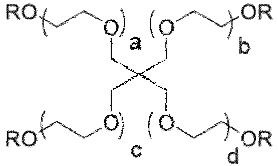 |
| R = | 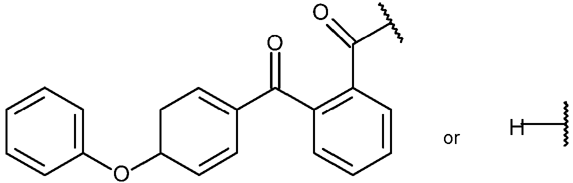 |
| | 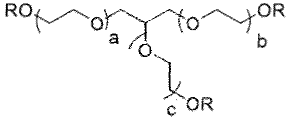 |
| R = | 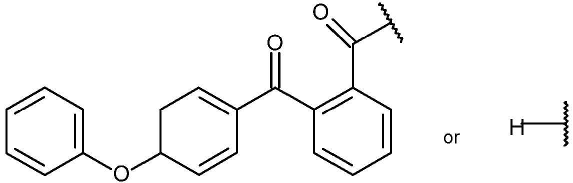 |
| | 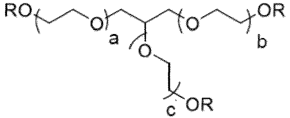 |
| R = | 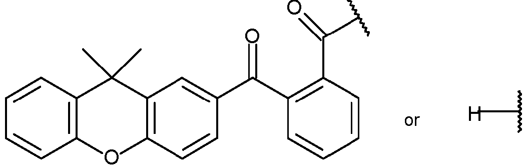 |
| | 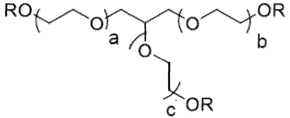 |
| R = | 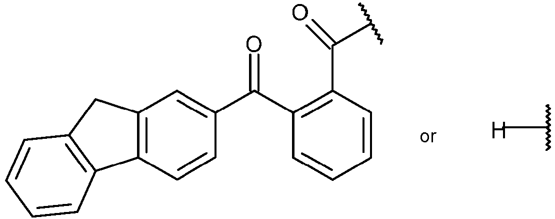 |
| | 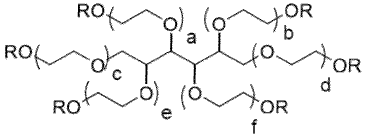 |
| R = | 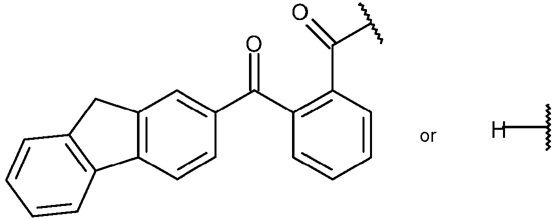 |
| | 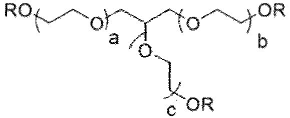 |
| R = | 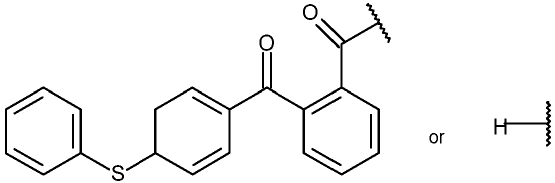 |
| | 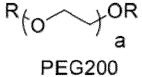 |
| R = | 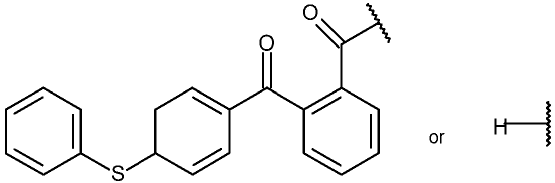 |
| | 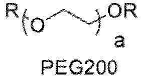 |
| R = | 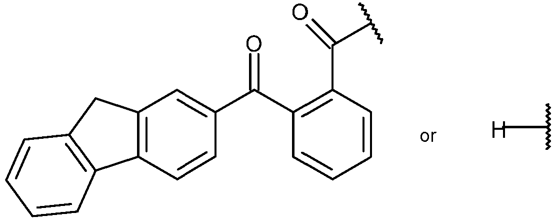 |
| | |
| | 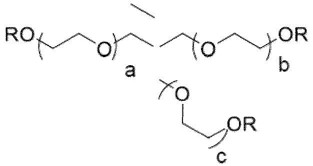 |
| R = | 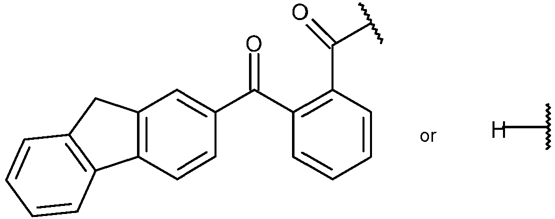 |
| | 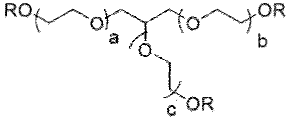 |
| R = | 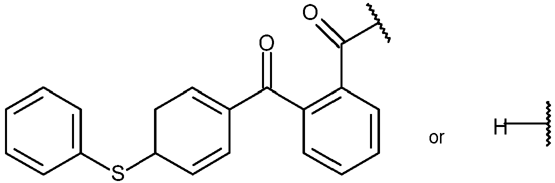 |
| | 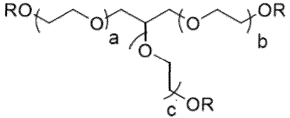 |
| R = | 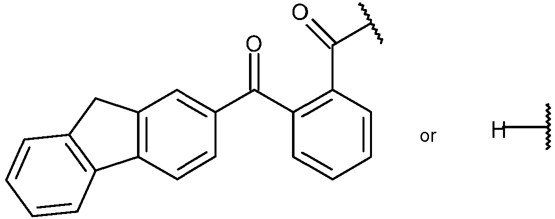 |

Letters (a) to (f) represent the number of ethoxy units and are, each independently, from 0 to 15 more preferably from 1 to 6.

Among the above compounds:
- those wherein Z is (I) and X is S; and
- those wherein Z is (III) and X is CH2;
are preferred.

According to another of its aspects, the present invention relates to a photopolymerizable composition comprising:
a) from 50 to 99.9%, preferably from 70 to 98.9% by weight, based on the total content of the composition, of at least one ethylenically unsaturated compound;
b) from 0.1 to 35%, preferably from 0.1 to 20%, and more preferably from 0.2 to 15% by weight, based on the total content of the composition, of at least one compound of formula (I) as above defined; and
c) from 0 to 20% by weight, preferably from 0 to 15%, and more preferably from 0.2 to 15% by weight, based on the total content of the composition, of an accelerator and/or of a coinitiator.

According to the present invention, the terms "photocuring" and "photopolymerizing" and related terms, are synonyms.

The expression "based on the total content of the composition" means that the weight % amounts of any of the components is calculated with respect to the sum of the weight of all the components of the composition, including any possible further additional components (in addition to a), b) and c) above), but possible water and/or solvents which may be present in the composition are not considered for the calculation of said weight % amounts.

According to another of its aspects, the present invention relates to a process for photocuring photopolymerizable compositions, coatings, adhesives and inks, which process comprises:
i. providing a photopolymerizable composition as above defined;
ii. coating or printing said photopolymerizable composition onto a substrate, and
iii. photocuring said coated or printed composition with a light source on said substrate.

According to another of its aspects, the present invention relates to a process for three-dimensional printing which comprising photocuring with a light source a mixture comprising a photopolymerizable composition as above defined.

According to another of its aspects, the present invention relates to an article of manufacture obtained by the process of the invention.

According to a preferred embodiment, the photopolymerizable composition used the processes of the invention comprises at least components (a), (b), (c), more preferably at least (a), (b), (c), and (d) as above defined. Compared to the prior art PIs, especially to those disclosed in WO2015/010729, the PIs of the invention showed a significant lower yellowing measured as Yellow Index, as it will be demonstrated in the Experimental Section which follows.

The photoinitiators of the invention can be used in photocurable compositions comprising ethylenically unsaturated compounds (a). Said unsaturated compounds (a) can contain one or more olefinic double bonds. They can be low-molecular weight (monomeric) or high-molecular weight (oligomeric) compounds.

Examples of suitable low molecular weight monomers (monomeric compounds) having one double bond are alkyl or hydroxyalkyl acrylates or methacrylates, such as methyl-, ethyl-, butyl-, 2-ethylhexyl-,2-hydroxyethyl- or isobornyl-acrylate; and methyl or ethyl methacrylate. Further examples are resins modified with silicon or fluorine, e.g. silicone acrylates. Further examples of these monomers are acrylonitrile, acrylamide, methacrylamide, N-substituted (meth)acrylamides, styrene, alkylstyrenes and halogeno styrenes, vinyl esters such as vinyl acetate, vinyl ethers such as iso-butyl vinyl ether, N-vinylpyrrolidone, vinyl chloride or vinylidene chloride.

Examples of monomers having more than one double bond are the ethylene glycol diacrylate, propylene glycol diacrylate, neopentyl glycol diacrylate, hexamethylene glycol diacrylate, bisphenol A diacrylate, 4,4'-bis-(2-acryloyloxyethoxy)-diphenylpropane, trimethylolpropane triacrylate, pentaerythritol triacrylate or tetraacrylate, vinyl acrylate, divinyl benzene, divinyl succinate, diallyl phthalate, triallyl phosphate, triallyl isocyanurate or tris-(2-acryloylethyl) isocyanurate.

Examples of high-molecular weight (oligomeric) polyunsaturated compounds are acrylated epoxy resins, acrylated or vinyl-ether- or epoxy-group-containing polyesters, acrylated polyurethanes or acrylated polyethers. Further examples of unsaturated oligomers are unsaturated polyester resins which are usually prepared from maleic acid, phthalic acid and one or more diols and which have molecular weights of from about 500 Da to 3,000 Da. Such unsaturated oligomers can also be referred to as prepolymers.

Examples of compounds (a) which are particularly suitable for the implementation of the present invention, are esters of ethylenically unsaturated carboxylic acids and polyols or polyepoxides, and polymers containing ethylenically unsaturated groups in the chain or in side groups, e.g. unsaturated polyesters, polyamides and polyurethanes and copolymers thereof, alkyl resins, polybutadiene and butadiene copolymers, polyisoprene and isoprene copolymers, polymers and copolymers having (meth)acrylic groups in side chains, as well as mixtures thereof.

Illustrative examples of unsaturated carboxylic acids or anhydrides, useful for the preparation of the above esters, are acrylic acid, methacrylic acid, maleic anhydride, crotonic acid, itaconic acid, cinnamic acid and unsaturated fatty acids such as linolenic acid and oleic acid. Acrylic and methacrylic acid are preferred.

Examples of polyols, which can also be esterified, are aromatic and aliphatic and cycloaliphatic polyols, preferably aliphatic and cycloaliphatic polyols.

Aromatic polyols are, for example, hydroquinone, 4,4'-dihydroxydiphenyl, 2,2-di(4-hydroxyphenyl) propane, as well as novolaks and resoles. Polyepoxides, which can be esterified, include those based on the said polyols, especially the reaction products between aromatic polyols and epichlorohydrin. Also suitable as polyols are polymers and copolymers that contain hydroxyl groups in the polymer chain or in side groups, for example polyvinyl alcohol and copolymers thereof or polymethacrylic acid hydroxyalkyl esters or copolymers thereof. Further suitable polyols are oligoesters carrying hydroxyl terminal groups.

Examples of aliphatic and cycloaliphatic polyols include alkylenediols containing preferably from 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene glycol, polyethylene glycols having molecular weights of preferably from 200 Da to 1,500 Da, 1,3-cyclopentanediol, 1,2-, 1,3- or 1,4-cyclohexanediol, 1,4-dihydroxymethyl cyclohexane, glycerol, tris(β-hydroxy-ethyl)amine, trimethylolethane, trimethylolpropane, pentaerythritol, dipentaerythritol and sorbitol.

Further suitable ethylenically unsaturated compounds (a) are unsaturated polyamides obtained from unsaturated carboxylic acids and aromatic, aliphatic and cycloaliphatic polyamines having preferably from 2 to 6, preferably from 2 to 4, amino groups. Examples of such polyamines are: ethylenediamine, 1,2- or 1,3-propylenediamine, 1,2-, 1,3- or 1,4-butylenediamine, 1,5-pentylenediamine, 1,6-hexylenediamine, octylenediamine, dodecylene diamine, 1,4-diaminocyclohexane, isophoronediamine, phenylene diamine, bisphenylenediamine, di-(β-aminoethyl) ether, diethylene triamine, triethylenetetramine and di(β-aminoethoxy)- and di(β-aminopropoxy)ethane. Other suitable polyamines are polymers and copolymers which may contain additional amino groups in the side chain and oligoamides containing amino end groups.

Specific examples of such unsaturated polyamides are methylenebisacrylamide, 1,6-hexamethylene bisacrylamide, diethylenetriamine trismethacrylamide, bis(methacrylamidopropoxy) ethane and N-[(β-hydroxyethoxy)ethyl]-acrylamide.

Unsaturated polyurethanes are also suitable for the implementation of the present invention as components (a), for example those derived from saturated or unsaturated diisocyanates and unsaturated or saturated diols. Polybutadiene and polyisoprene and copolymers thereof may also be used. Suitable monomers include, for example, olefins, such as ethylene, propene, butene and hexene, (meth)acrylates, acrylonitrile, styrene and vinyl chloride.

Polymers having unsaturated (meth)acrylate groups in the side chain can also be used as component (a). They may typically be reaction products of epoxy resins based on novolac with (meth)acrylic acid; homo- or copolymers of vinyl alcohol or hydroxyalkyl derivatives thereof that have been esterified with (meth)acrylic acid; and homo- and co-polymers of (meth)acrylates that have been esterified with hydroxyalkyl (meth)acrylates.

According to a preferred embodiment, the photocurable composition further comprises a coinitiators (c), also referred to as accelerators. Suitable examples of accelerators/coinitiators (c) are alcohols, thiols, thioethers, amines or ethers that have an available hydrogen, bonded to a carbon adjacent to the heteroatom, disulfides and phosphines, e.g. as described in EP 438 123 and GB 2 180 358.

Suitable examples of amine accelerators/co-initiators include, but are not limited to, aliphatic, cycloaliphatic, aromatic, aryl-aliphatic, heterocyclic, oligomeric or polymeric amines. They can be primary, secondary or tertiary amines, for example butyl amine, dibutyl amine, tributyl amine, cyclohexyl amine, benzyldimethyl amine, di-cyclohexyl amine, N-phenyl glycine, triethyl amine, phenyl-diethanol amine, triethanolamine, piperidine, piperazine, morpholine, pyridine, quinoline, esters of dimethylamino benzoic acid, Michler's ketone (4,4'-bis-dimethyl aminobenzophenone) and derivatives thereof.

As the amine accelerators/co-initiators, an amine-modified acrylate compound can be used; examples of such amine-modified acrylate include acrylates modified by reaction with a primary or secondary amine that are described in US 3,844,916, EP 280222, US 5,482,649 or US 5,734,002. Multifunctional amine and polymeric amine derivatives are also suitable as co-initiators some examples are those presently marketed as Omnipol^{®} ASA from IGM Resins B.V., Genopol^{®} AB-2 from Rahn A.G., Speedcure^{®} 7040 from Lambson Limited or those described in US2013/0012611.

The photocurable compositions of the present invention can also comprise one or more of the following components: (d) photosensitizers and/or (e) further photoinitiators and/or (f) conventional additives, in addition to compounds (a), (b) and, when present, (c).

The photocurable compositions of the present invention can also be formulated in compositions further comprising water and/or solvents, such as organic solvents.

Photosensitizers (d) can be present in an amount comprised between 0.01 and 15% by weight, based on the total content of the composition, preferably between 0.01 and 10% by weight.

Examples of photosensitizers are those commonly used in the art, aromatic carbonyl compounds, e.g. benzophenones, thioxanthones, anthraquinones, coumarins and 3-acylcoumarin derivatives, terphenyls, styryl ketones, and 3-(aroylmethylene)-thiazolines, camphorquinones and also eosin, rhodamine and erythrosine dyes.

Examples of thioxanthones are thioxanthone, 2-isopropyl thioxanthone, 2-chloro thioxanthone, 2-dodecyl thioxanthone, 2,4-diethyl thioxanthone, 2,4-dimethyl thioxanthone, 1-methoxycarbonyl thioxanthone, 2-ethoxycarbonyl thioxanthone, 3-(2-methoxyethoxycarbonyl) thioxanthone, 4-butoxycarbonyl thioxanthone, 3-butoxycarbonyl-7-methyl thioxanthone, 1-cyano-3-chloro thioxanthone, 1-ethoxycarbonyl-3-chloro thioxanthone, 1-ethoxycarbonyl-3-ethoxy thioxanthone, 1-ethoxycarbonyl-3-amino thioxanthone, 1-ethoxycarbonyl-3-phenylsulfuryl thioxanthone, 3,4-di[2-(2-methoxyethoxy)ethoxycarbonyl] thioxanthone, 1-ethoxycarbonyl-3-(1-methyl-1-morpholinoethyl) thioxanthone, 2-methyl-6-dimethoxymethyl thioxanthone, 2-methyl-6-(1,1-dimethoxybenzyl) thioxanthone, 2-morpholinomethyl thioxanthone, 2-methyl-6-morpholinomethyl thioxanthone, N-allylthioxanthone-3,4-dicarboximide, N-octylthioxanthone-3,4-dicarboximide, N-(1,1,3,3-tetramethylbutyl)-thioxanthone-3,4-dicarboximide, 1-phenoxy thioxanthone, 6-ethoxycarbonyl-1-2-methoxythioxanthone, 6-ethoxycarbonyl-2-methylthioxanthone, thioxanthone-2-polyethylene glycol ester, 2-hydroxy-3-(3,4-dimethyl-9-oxo-9H-thioxanthon-2-yloxy)-N,N,N-trimethyl-1-propanaminium chloride, or those described in the patent application PCT/EP2011/069514, such as n-dodecyl-7-methyl-thioxanthone-3-carboxylate and N,N-disobutyl-7-methyl-thioxanthone-3-carbamide. Also suitable are polymeric thioxanthone derivatives (e.g. those presently marketed as Omnipol^{®} TX from IGM Resins B.V., Genopol^{®} TX-1 from Rahn A.G., Speedcure^{®} 7010 from Lambson Limited). Example of benzophenones are benzophenone, 4-phenyl benzophenone, 4-methoxy benzophenone, 4,4'-dimethoxybenzophenone, 4,4'-dimethyl benzophenone, 4,4'-dichloro benzophenone, 4,4'-dimethylamino benzophenone, 4,4'-diethylamino benzophenone, 4-methyl benzophenone, 2,4,6-trimethyl benzophenone, 4-(4-methylthiophenyl) benzophenone, 3,3'-dimethyl-4-methoxy benzophenone, methyl 2-benzoyl benzoate, 4-(2-hydroxyethylthio) benzophenone, 4-(4-tolylthio) benzophenone, 4-benzoyl-N,N,N-trimethylbenzene methanaminium chloride, 2-hydroxy-3-(4-benzoylphenoxy)-N,N,N-trimethyl-1-propanaminium chloride monohydrate, 4-(13-acryloyl-1,4,7,10,13-pentaoxatridecyl) benzophenone, 4-benzoyl-N,N-dimethyl-N-[2-(1-oxo-2-propenyl)oxylethyl-benzene methanaminium chloride, or those described in US9938231 (e.g. Omnirad^{®} 991 from IGM Resins B.V.).

Also suitable are polymeric benzophenone derivatives (e.g. those presently marketed as Omnipol^{®} BP, Omnipol^{®} 2702 and Omnipol^{®} 682 all from IGM Resins B.V., Genopol^{®} BP-2 from Rahn A.G. and Speedcure^{®} 7005 from Lambson Limited).

Examples of 3-acylcoumarin derivatives are 3-benzoyl coumarin, 3-benzoyl-7-methoxy coumarin, 3-benzoyl-5,7-di(propoxy) coumarin, 3-benzoyl-6,8-dichloro coumarin, 3-benzoyl-6-chloro coumarin, 3,3'-carbonyl-bis[5,7-di(propoxy) coumarin], 3,3'-carbonyl-bis(7-methoxy coumarin), 3,3'-carbonyl-bis(7-diethylamino coumarin), 3-isobutyroyl coumarin, 3-benzoyl-5,7-dimethoxy coumarin, 3-benzoyl-5,7-diethoxy coumarin, 3-benzoyl-5,7-dibutoxy coumarin, 3-benzoyl-5,7-di(methoxyethoxy) coumarin, 3-benzoyl-5,7-di(allyloxy) coumarin, 3-benzoyl-7-dimethylamino coumarin, 3-benzoyl-7-diethylamino coumarin, 3-isobutyroyl-1,7-dimethylamino coumarin, 5,7-dimethoxy-3-(1-benzoyl) coumarin, 5,7-dimethoxy-3(1-benzoyl)-coumarin, 3-benzoylbenzo [f]coumarin, 7-diethylamino-3-thienoyl coumarin, 3-(4-cyanobenzoyl)-5,7-dimethoxy coumarin, or those described in EP2909243 and WO2017216699.

Examples of 3-(aroylmethylene) thiazolines are 3-methy-1,2-benzoylmethylene-β-benzo thiazoline, 3-methyl-2-benzoylmethylene-benzo thiazoline, 3-ethyl-2-propionylmethylene-β-benzo thiazoline.

Examples of other aromatic carbonyl compounds are acetophenone, 3-methoxyacetophenone, 4-phenylacetophenone, benzil, such as that described in WO 2013/164394, 2-acetylnaphthalene, 2-naphthaldehyde, 9,10-anthraquinone, 9-fluorenone, dibenzosuberone, xanthone, 2,5-bis(4-diethylaminobenzylidene) cyclopentanone, α-(para-dimethylamino benzylidene), ketones, such as 2-(4-dimethylamino-benzylidene)-indan-1-one or 3-(4-dimethylaminophenyl)-1-indan-5-yl-propenone, 3-phenylthiophthalimide, N-methyl-3,5-di(ethylthio) phthalimide.

Particularly preferred are thioxanthones, coumarins and 3-acylcoumarins. It was observed that the above components (d) increase the activity of photoinitiators (b) without shortening the shelf life of the compositions. Moreover, such compositions have the special advantage that an appropriate choice of the photosensitizer (d) allows the spectral sensitivity of photoinitioator (b) to be shifted to any desired wavelength region. The skilled in the art is able to select the suitable photosensitizer (d) to make the photoinitiator(s) (b) work at any desired wavelength region.

The further possible photoinitiators (e) can be present in an amount comprised between 0.5 and 15 % by weight, of the total content of the composition, preferably between 1 and 10% by weight of the composition. Examples of other suitable photoinitiators (e) are camphorquinone, benzophenone, benzophenone derivatives, acetophenone, acetophenone derivatives, dialkoxyacetophenones, α-hydroxyketones, α-aminoketones, 4-aroyl-1,3-dioxolanes, benzoin alkyl ethers and benzil ketals, e.g. benzil dimethyl ketal, ketosulfones, e.g 1-[4-[(4-benzoyl-phenyl)-thio]-phenyl]-2-methyl-2-[(4-methyl-phenyl)-sulfonyl]-propan-1-one (Esacure^{®} 1001, from IGM Resins B.V.), 3-ketocoumarins, for example as described in EP2909243 and WO2017216699, phenylglyoxylates and derivatives thereof, dimeric phenyl glyoxylates, peresters, e.g. benzophenonetetracarboxylic acid peresters, for example as described in EP 126 541, acylphosphine photoinitiators (which can be selected from mono-acylphosphine oxides, bis-acylphosphine oxides, tris-acylphosphine oxides and multifunctional mono- or bisacylphosphine oxides), halomethyltriazines, hexaaryl bisimidazole/coinitiator systems, e.g. ortho-chlorohexaphenylbisimidazole in combination with 2-mercaptobenzothiazole, ferrocenium compounds or titanocenes, for example dicyclopentadienyl-bis(2,6-difluoro-3-pyrrolo-phenyl)titanium, O-acyloxime ester photoinitiators.

Examples of α-hydroxyketones and α-aminoketones are 1-hydroxy cyclohexylphenyl ketone, 2-hydroxy-2-methyl-1-phenyl-propane-1-one, 1-[4-(2-hydroxyethoxy)phenyl]-2-hydroxy-2-methyl-1-propane-1-one, 2-hydroxy-1-{4-[4-(2-hydroxy-2-methyl-propionyl)-benzyl]phenyl}-2-methyl-propane-1-one), 2-hydroxy-1-{4-[4-(2-hydroxy-2-methylpropionyl)-phenoxy]-phenyl}-2-methyl-propan-1-one, 2-methyl-1-(4-methylthiophenyl)-2-morpholinopropane-1-one), 2-benzyl-2-dimethylamino-1-(4-morpholinophenyl)-butane-1-one, and (2-(dimethylamino)-2-[(4-methylphenyl)methyl]-1-[4-(4-morpholinyl) phenyl]-1-butanone).

Examples of O-acyloxime ester photoinitiators are 1,2-octanedione,1-[4-(phenylthio)phenyl]-2-(O-benzoyloxime), ethanone 1-[9-ethyl-6-(2-methylbenzoyl)-9H-carbazole-3-yl] 1-(O-acetyloxime) or those described in GB 2339571.

Examples of acylphosphine photoinitiators include, but are not limited to, bis(2,4,6-trimethylbenzoyl)phenylphosphine oxide, bis(2,6-dimethoxybenzoyl)-2,4,4-trimethylpentyl phosphine oxide, bis(2,4,6-trimethylbenzoyl)-(2,4-dipentyloxyphenyl), 2,4,6-trimethylbenzoyl-diphenyl phosphine oxide, ethyl (2,4,6-trimethylbenzoyl) phenylphosphinate, and phenyl(2,4,6-trimethylbenzoyl)phosphinic acid glycerol ethoxylated triester (Omnipol TP from IGM Resins B.V.).

Examples of the halomethyltriazines based photoinitiators are 2-[2-(4-methoxy-phenyl)-vinyl]-4,6-bis-trichloromethyl [1,3,5]triazine, 2-(4-methoxy-phenyl)-4,6-bis-trichloromethyl [1,3,5]triazine, 2-(3,4-dimethoxyphenyl)-4,6-bis-trichloromethyl [1,3,5]triazine, 2-methyl-4,6-bis-trichloromethyl [1,3,5] triazine.

Cationic photoinitiators can be also used as the further photoinitiators (e), when the photocurable compositions according to the invention are used in hybrid systems (which in this connection mean mixtures of free- radically and cationically curing systems). Examples of suitable cationic photoinitiators are aromatic sulfonium, phosphonium or iodonium salts, as described e.g. in US4,950,581, or cyclopentadienylarene-iron(II) complex salts, e.g. (η⁶-isopropylbenzene)(η⁵-cyclopentadienyl) iron(II) hexafluorophosphate or photolatent acids based on oximes, as described, for example, in GB 2 348 644, US4,450,598, US4,136,055, WO 00/10972 and WO 00/26219.

The photocuring composition according to the invention may also comprise conventional additives, from 0 to 10% based on the total content of the composition. Additives (f) can be, for example, thermal initiators, binders, stabilizers, and mixture thereof.

The choice of additives is governed by the field of use in question and the properties desired for that field. The additives (f) described above are known in the art and are accordingly used in the amounts conventionally used in the art.

For instance, especially in the case of pigmented compositions, the composition may also comprise, as additional additive (f), a thermal initiator, a compound that forms free radicals when heated, e.g. an azo compounds, such as 2,2'-azobis(4-methoxy-2,4-dimethylvaleronitrile), a triazene, diazosulfide, pentazadiene or a peroxy compound, for example a hydroperoxide or peroxycarbonate, e.g. tert-butyl hydroperoxide, as described e.g. in EP 245 639.

Binders may also be added to the photocurable composition of the invention. The addition of binders is particularly advantageous when the photocurable compounds are liquid or viscous substances. The amount of binder may be, for example, from 5 to 60% by weight, preferably from 10 to 50% by weight, based on the total content of the composition, excluding possible water and solvents. The choice of binder is made in accordance with the field of use and the properties required therefor, such as developability in aqueous and organic solvent systems, adhesion to substrates and sensitivity to oxygen.

Suitable binders are, for example, polymers having a weight average molecular weight (Mw) of approximately from 5,000 Da to 2,000,000 Da, preferably from 10,000 Da to 1,000,000 Da. Illustrative examples are: homo- and copolymers of acrylates and methacrylates, e.g. copolymers of methyl methacrylate/ethyl acrylate/methacrylic acid, poly(methacrylic acid alkyl esters), poly(acrylic acid alkyl esters); cellulose esters and ethers, such as cellulose acetate, cellulose acetate butyrate, methylcellulose, ethylcellulose, polyvinylbutyral, polyvinylformal, cyclised rubber, polyethers such as polyethylene oxide, polypropylene oxide, polytetrahydrofuran, polystyrene, polycarbonates, polyurethanes, chlorinated polyolefins, e.g. polyvinyl chloride, co-polymers of vinyl chloride/vinylidene chloride, co-polymers of vinylidene chloride with acrylonitrile, methyl methacrylate and vinyl acetate, polyvinyl acetate, co-poly (ethylene/vinyl acetate), polymers such as polycaprolactam and poly(hexamethylene adipamide), polyesters such as poly(ethylene glycol terephthalate) and poly(hexamethylene glycol succinate).

Suitable stabilizers are, for example, thermal inhibitors, such as hydroquinone, hydroquinone derivatives, p-methoxyphenol, β-benzol or sterically hindered phenols, e.g. 2,6-di(tert-butyl)-p-cresol, which prevent premature polymerization. In order to increase dark storage stability it is possible to use, for example, copper compounds, such as copper naphthenate, stearate or octoate, phosphorus compounds, for example triphenylphosphine, tributylphosphine, triethyl phosphite, triphenyl phosphite or tribenzyl phosphite, quaternary ammonium compounds, e.g. tetramethylammonium chloride or trimethylbenzylammonium chloride, or hydroxylamine derivatives, e.g. N,N-diethylhydroxylamine. For the purpose of excluding atmospheric oxygen during polymerization it is possible to add paraffin or similar wax-like substances which, being insoluble in the polymer, migrate to the surface at the beginning of the polymerization and form a transparent surface layer which prevents air from entering.

It is also possible to add a light stabilizer, such as UV absorbers, e.g. hydroxyphenylbenzotriazole, hydroxyphenylbenzophenone, oxalic acid amide or hydroxyphenyl-s-triazine type. Such components can be used on their own or in the form of mixtures, with or without the use of sterically hindered amines (HALS).

The photocurable compositions according to the invention may also comprise, as further additives (f), photoreducible dyes, e.g. a xanthene, benzoxanthene, benzothioxanthene, thiazine, pyronin, porphyrin or acridine dye, and/or radiation cleavable trihalomethyl compounds. These compounds are described, for example, in EP445624.

Further customary additives (f) are, depending upon the intended use, optical brighteners, fillers, pigments, both white and colored pigments, colorants, antistatics, wetting agents or flow improvers. Additives conventionally used in the art, e.g. antistatics, flow improvers and adhesion enhancers, can also be used.

In addition to the above components, other components may be present in the composition of the invention.

It is also possible for chain-transfer reagents conventionally used in the art to be added to the compositions according to the invention. Examples are mercaptans, amines and benzothiazole.

The composition of the invention may also comprise colorants and/or colored pigments. Depending upon the intended use, both inorganic and organic pigments may be used. Such additives are well known to the person skilled in the art; some examples are carbon black, iron oxides, such as iron oxide yellow, iron oxide red, chromium yellow, chromium green, nickel titanium yellow, ultramarine blue, cobalt blue, bismuth vanadate, cadmium yellow and cadmium red. Examples of organic pigments are mono- or bis-azo pigments, and also metal complexes thereof, phthalocyanine pigments, polycyclic pigments, e.g. perylene, anthraquinone, thioindigo, quinacridone or triphenylmethane pigments, and also diketo-pyrrolo-pyrrole, isoindolinone, e.g. tetrachloroisoindolinone, isoindoline, dioxazine, benzimidazolone and quinophthalone pigments. The pigments may be used in the formulations on their own or in admixture.

Depending upon the intended use, the pigments can be added to the formulations in amounts conventionally used in the art, for example in an amount from 0.1 to 30% by weight or from 10 to 25% by weight, based on the total weight of the composition.

The composition may also comprise, for example, organic colorants of an extremely wide variety of classes. Examples are azo dyes, methine dyes, anthraquinone dyes and metal complex dyes. Usual concentrations are, for example, from 0.1 to 20% wt, especially from 1 to 5% wt, based on the total weight of the composition.

The photocurable compositions of the invention may comprise water.

The photocurable compositions of the invention are suitable for various purposes, for example as a printing ink, such as screen printing inks, flexographic printing inks, offset printing inks and inkjet printing inks, as clearcoats, as colored coats, for example for wood or metal, as powder coatings, as coating materials inter alia for paper, wood, metal or plastics, as daylight-curable paints for marking structures and roads, for photographic reproduction processes, for holographic recording materials, for image-recording processes or in the production of printing plates that can be developed using organic solvents or using aqueous-alkaline media, for the production of masks for screen printing, as dental filling compounds, as adhesives, as pressure-sensitive adhesives, as laminating resins, as photoresists, e.g. galvanoresists, as etch resists or permanent resists, both liquid and dry films, as photostructurable dielectrics, and as solder masks for electronic circuits, as resists in the production of color filters for any type of display screen or in the creation of structures during the manufacture of plasma displays and electroluminescent displays, in the production of optical switches, optical gratings (interference gratings), in the manufacture of three-dimensional articles by bulk curing (UV curing in transparent moulds) or according to the stereolithography process, as described, for example, in US4,575,330, in the manufacture of composite materials (e.g. styrene polyesters which may include glass fibers and/or other fibers and other adjuvants) and other methods of printing in three dimensions well-known to one skilled in the art, in the coating or sealing of electronic components or as coatings for optical fibers.

The photocurable compositions of the invention are also suitable for the production of optical lenses, e.g. contact lenses or Fresnel lenses, in the manufacture of medical apparatus, aids or implants, in dry film paints. The photocurable compositions of the invention are also suitable for the preparation of gels having thermotropic properties. Such gels are described for example in DE 19700064 and EP 678534.

An article comprising a compound of formula (I), or comprising a photocurable composition of the invention, represents a further subject-matter of the invention.

The compounds and compositions according to the invention may also be used as free-radical photoinitiators or photoinitiating systems for radiation-curable powder coatings.

The photocurable compositions according to the invention are suitable, for example, as coating materials for all kinds of substrate, for example wood, textiles, paper, ceramics, glass, plastics, such as polyesters, polyethylene terephthalate, polyolefins and cellulose acetate, especially in the form of films, and also metals, such as Al, Cu, Ni, Fe, Zn, Mg or Co and GaAs, Si or SiO₂, to which a protective layer is to be applied or an image is to be applied e.g. by imagewise exposure.

A large number of the most varied kinds of light source may be used in the process according to the invention, the light source emitting at wavelengths from approximately 200 nm to approximately 800 nm. Both point sources and planiform radiators (lamp carpets) are suitable. Examples are: carbon arc lamps, xenon arc lamps, medium pressure, high pressure and low pressure mercury arc radiators, doped, where appropriate, with metal halides (metal halide lamps), microwave-excited metal vapour lamps, excimer lamps, superactinic fluorescent tubes, fluorescent lamps, argon incandescent lamps, flash lamps, photographic floodlight lamps, light-emitting diodes (LED), electron beams, X-rays and lasers.

According to one embodiment, said light source comprises UV light in at least one of the UVA, UVB and UVC ranges.

According to a preferred embodiment, said light source is a LED source, particularly preferred are LED light source emitting at wavelengths comprised between 365 nm and 420 nm, more preferably at 365 nm, 385 nm and 395 nm.

According to the invention the distance between the lamp and the substrate to be exposed may vary according to the intended use and the type and strength of the lamp, e.g. from 0.1 cm to 150 cm, preferably from 1 cm to 50 cm.

Said photopolymerizable composition may also be applied over a substrate already comprising a coated or printed layer. Said photopolymerizable composition may, after photopolymerization with said light source, be overprinted or overcoated with one or more compositions suitable for printing or coating.

The article obtained by applying said photopolymerizable composition to said substrate by said means of coating or printing, and photopolymerizing by said light source, with or without further elaboration of the article by further coating or printing, represents a further subject-matter of this invention.

As said, we surprisingly found that compounds of formula (I) have a very high reactivity under UVA, UVB and UVC wavelength maintaining a low post cure yellowing compared to that of the prior art. The new compounds showed their great improvement in surface curing under LED and Hg lamps both in clear and pigmented systems.

The invention is illustrated in detail below by the following examples, which are illustrative and not limiting.

### Experimental Section

In case of inconsistencies between the chemical name and the chemical structure herein indicated, the chemical structure prevails.

¹H NMR spectra were recorded with a Bruker Ascend 300 MHz NMR Spectrometer.

Letters (a) to (f) represent the number of ethoxy units.

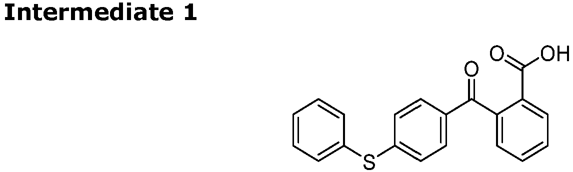

To an ice-cooled mixture of 5.00 g (26.843 mmoles) of diphenyl sulfide and 3.98 g (26.870 mmoles) of phthalic anhydride in 90 mL of chlorobenzene, 7.15 g (53.622 mmoles) of anhydrous aluminum chloride were cautiously added in portions. The reaction mixture was stirred at room temperature for 1.5 hours and then poured into ice/dilute hydrochloric acid. The mixture was extracted with ethyl acetate and the organic layer was separated, washed with 1M hydrochloric acid, dried over anhydrous sodium sulfate, filtered and the solvent removed by distillation under vacuum obtaining the crude. The obtained crude was purified by crystallization from toluene obtaining 6.55 g of a white solid (yield 73%). ¹H-NMR (DMSO-d₆, δ ppm): 7.24 (d, 2H), 7.40 (dd, 1H), 7.46-7.56 (m, 7H), 7.61-7.74 (m, 2H), 7.99 (dd, 1H).

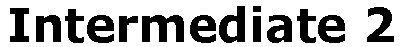

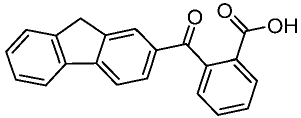

To an ice-cooled mixture of 15.00 g (90.242 mmoles) of fluorene and 13.36 g (90.197 mmoles) of phthalic anhydride in 270 mL of chlorobenzene, 24.07 g (180.516 mmoles) of anhydrous aluminum chloride were cautiously added in portions. The reaction mixture was stirred at room temperature for 1.5 hours and then poured into ice/dilute hydrochloric acid. The formed semisolid product was treated with 270 mL of ethyl acetate, and the obtained solid was filtered off. The filtrate was made to separate and the organic layer was washed with 1M hydrochloric acid, dried over anhydrous sodium sulfate, filtered and the solvent removed by distillation under vacuum obtaining the crude. The obtained crude was joined to the previously filtered solid, suspended in 200 mL of toluene and washed under reflux and vigorous stirring for 30 minutes. Then the mixture was allowed to cool and filtered off obtaining 27.26 g of a white solid (yield 96%).

¹H-NMR (DMSO-d₆, δ ppm): 3.99 (s, 2H), 7.38-7.48 (m, 3H), 7.60-7.76 (m, 4H), 7.86 (s, 1H), 7.98-8.04 (m, 3H).

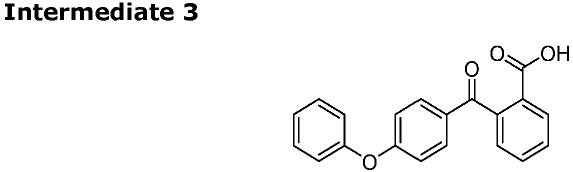

To an ice-cooled mixture of 15.00 g (88.126 mmoles) of diphenyl ether and 13.05 g (88.104 mmoles) of phthalic anhydride in 250 mL of chlorobenzene, 23.50 g (176.241 mmoles) of anhydrous aluminum chloride were cautiously added in portions. The reaction mixture was stirred at room temperature for 1.5 hours and then poured into ice/dilute hydrochloric acid. The mixture was extracted with ethyl acetate and the organic layer was separated, washed with 1M hydrochloric acid, dried over anhydrous sodium sulfate, filtered and the solvent removed by distillation under vacuum obtaining the crude. The obtained crude was purified by crystallization from toluene obtaining 23.25 g of a white solid (yield 83%). ¹H-NMR (DMSO-d₆, δ ppm): 7.03 (d, 2H), 7.14 (m, 2H), 7.25 (m, 1H), 7.38-7.49 (m, 3H), 7.62-7.75 (m, 4H), 7.98 (dd, 1H).

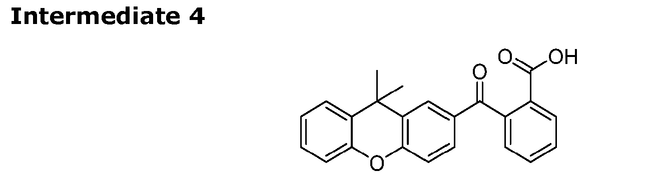

To an ice-cooled mixture of 5.15 g (24.491 mmoles) of 9,9-dimethylxanthene and 3.63 g (24.507 mmoles) of phthalic anhydride in 85 mL of chlorobenzene, 6.53 g (48.973 mmoles) of anhydrous aluminum chloride were cautiously added in portions. The reaction mixture was stirred at room temperature for 1.5 hours and then poured into ice/dilute hydrochloric acid. The mixture was extracted with ethyl acetate and the organic layer was separated, washed with 1M hydrochloric acid, dried over anhydrous sodium sulfate, filtered and the solvent removed by distillation under vacuum obtaining the crude. The obtained crude was purified by crystallization from toluene obtaining 7.15 g of a white solid (yield 81%). ¹H-NMR (DMSO-d₆, δ ppm): 1.58 (s, 6H), 7.09-7.20 (m, 3H), 7.28 (m, 1H), 7.38-7.46 (m, 2H), 7.58 (dd, 1H), 7.62-7.78 (m, 2H), 7.93 (d, 1H), 8.00 (dd, 1H).

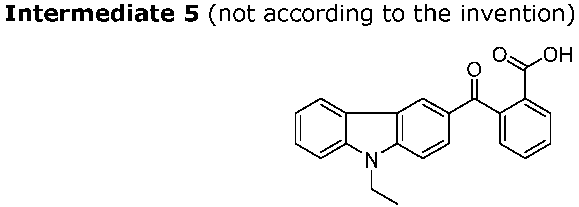

To an ice-cooled mixture of 15.00 g (76.817 mmoles) of 9-ethylcarbazole and 5.69 g (38.415 mmoles) of phthalic anhydride in 250 mL of chlorobenzene, 5.12 g (38.398 mmoles) of anhydrous aluminum chloride were cautiously added in portions. The reaction mixture was stirred at room temperature for 1 hour, then the reaction was cooled again and additional 2.84 g (19.174 mmoles) of phthalic anhydride and 2.56 g (19.199 mmoles) of anhydrous aluminum chloride were added in sequence. The reaction mixture was stirred at room temperature for further 1 hour, then the reaction was cooled again and additional 1.42 g (9.587 mmoles) of phthalic anhydride and 1.28 g (9.600 mmoles) of anhydrous aluminum chloride were added in sequence. The reaction mixture was stirred at room temperature for further 1 hour and then poured into ice/dilute hydrochloric acid. The mixture was extracted with ethyl acetate and the organic layer was separated, washed with 1M hydrochloric acid, dried over anhydrous sodium sulfate, filtered and the solvent removed by distillation under vacuum obtaining the crude. The obtained crude was suspended in 250 mL of toluene and washed under reflux and vigorous stirring for 30 minutes. Then the mixture was allowed to cool and the solid filtered off. The obtained solid was suspended in 200 mL of toluene and washed again under reflux and vigorous stirring for 24 hours. Then the mixture was allowed to cool and filtered off obtaining 7.61 g of a white solid (yield 33%).

¹H-NMR (DMSO-d₆, δ ppm): 1.34 (t, 3H), 4.49 (q, 2H), 7.23 (t, 1H), 7.44-7.55 (m, 2H), 7.64-7.77 (m, 5H), 8.01 (dd, 1H), 8.20 (d, 1H), 8.50 (d, 1H).

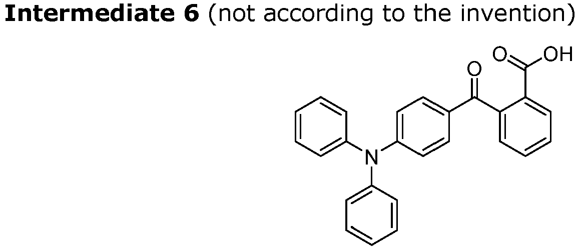

To an ice-cooled mixture of 15.00 g (61.145 mmoles) of triphenylamine and 4.53 g (30.583 mmoles) of phthalic anhydride in 200 mL of chlorobenzene, 4.07 g (30.523 mmoles) of anhydrous aluminum chloride were cautiously added in portions. The reaction mixture was stirred at room temperature for 1 hour, then the reaction was cooled again and additional 2.27 g (15.325 mmoles) of phthalic anhydride and 2.04 g (15.299 mmoles) of anhydrous aluminum chloride were added in sequence. The reaction mixture was stirred at room temperature for further 1 hour, then the reaction was cooled again and additional 1.89 g (12.760 mmoles) of phthalic anhydride and 1.70 g (12.749 mmoles) of anhydrous aluminum chloride were added in sequence. The reaction mixture was stirred at room temperature for further 1 hour and then poured into 400 mL of ice/water. The mixture was extracted with ethyl acetate and the organic layer was separated, dried over anhydrous sodium sulfate, filtered and the solvent removed by distillation under vacuum obtaining the crude. The crude product was purified by flash column chromatography on silica gel (toluene/ethyl acetate 50:50) obtaining 7.85 g of a yellow solid (yield 34%).

¹H-NMR (DMSO-d₆, δ ppm): 6.83 (d, 2H), 7.10-7.28 (m, 6H), 7.33-7.43 (m, 5H), 7.49 (d, 2H), 7.56-7.71 (m, 2H), 7.97 (dd, 1H).

### Example 1

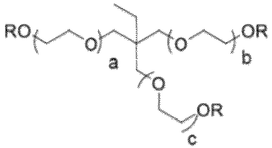

R =

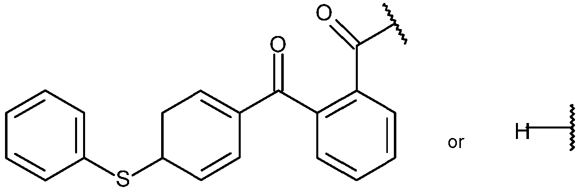

3.00 g (8.972 mmoles) of Intermediate 1, 1.52 g (hydroxyl n°364/g) of polyhol 3380 (purchased from PERSTORP) and 0.171 g (0.899 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.35 g of a colorless oil (yield 100%).

¹H-NMR (DMSO-d₆, δ ppm): 0.70 (m, 3H), 1.18 (m, 2H), 3.00-3.70 (m, 33H), 3.85-4.35 (m, 5H), 7.10-7.32 (m, 5H), 7.32-7.60 (m, 20H), 7.60-7.85 (m, 5H), 7.85-8.02 (m, 2.5H).

### Example 2

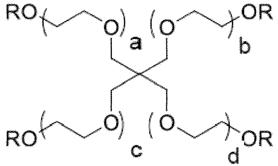

R =

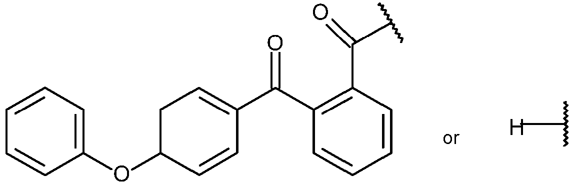

3.00 g (9.424 mmoles) of Intermediate 3, 0.918 g (hydroxyl n°634/g) of polyol 4640 (purchased from Perstorp) and 0.179 g (0.941 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 3.74 g of a pale oil (yield 100%).

¹H-NMR (DMSO-d₆, δ ppm): 2.98-3.70 (m, 5.7H), 3.81-4.35 (m, 2H), 6.80-7.28 (m, 5H), 7.30-7.80 (m, 7H), 7.80-8.06 (m, 1H).

### Example 3

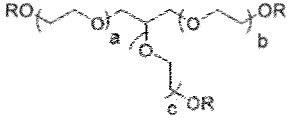

R =

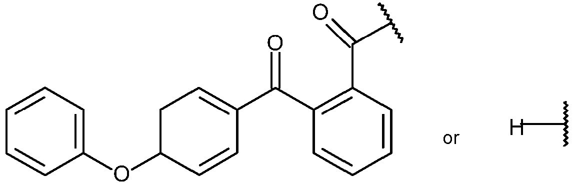

3.00 g (9.424 mmoles) of Intermediate 3, 1.56 g (hydroxyl n°370/g) of AIONICO GL/609 (purchased from Lamberti SpA) and 0.179 g (0.941 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.39 g of a pale oil (yield 100%).

¹H-NMR (DMSO-d₆, δ ppm): 3.03-3.78 (m, 11.45H), 4.00-4.38 (m, 2H), 6.91-7.20 (m, 4H), 7.32-7.57 (m, 4H), 7.57-7.86 (m, 4H), 7.86-8.03 (m, 1H).

### Example 4

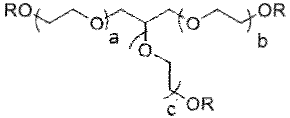

R =

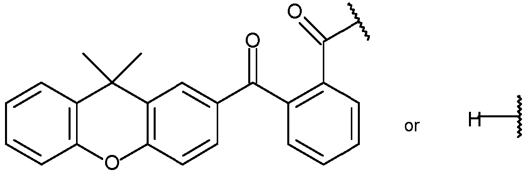

3.00 g (8.371 mmoles) of Intermediate 4, 1.39 g (hydroxyl n°370/g) of AIONICO GL/609 (purchased from Lamberti SpA) and 0.159 g (0.836 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.24 g of a pale oil (yield 100%).

¹H-NMR (DMSO-d₆, δ ppm): 1.60 (m, 6H), 3.05-3.75 (m, 11.5H), 3.91-4.30 (m, 2H), 7.00-7.32 (m, 3H), 7.36-7.60 (m, 3H), 7.60-7.85 (m, 3H), 7.88-8.03 (m, 2H).

### Example 5

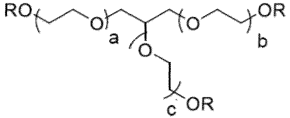

R =

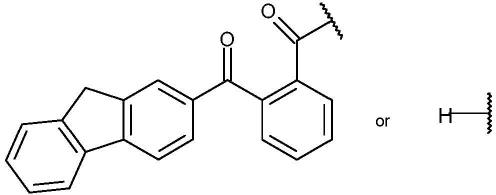

3.00 g (9.544 mmoles) of Intermediate 2, 1.66 g (hydroxyl n°370/g) of AIONICO GL/609 (purchased from Lamberti SpA) and 0.182 g (0.957 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.49 g of a pale oil (yield 100%). ¹H-NMR (DMSO-d₆, δ ppm): 3.05-3.78 (m, 10.15H), 3.79-4.35 (m, 4H), 7.28-7.81 (m, 7H), 7.81-8.08 (m, 4H).

### Example 6 (not according to the invention)

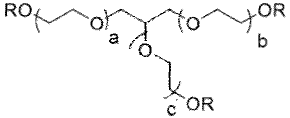

R =

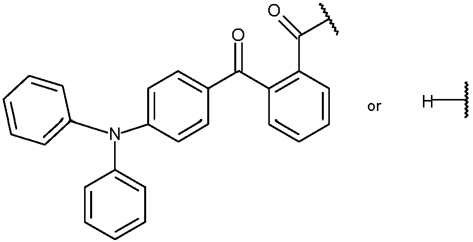

3.00 g (7.625 mmoles) of Intermediate 6, 1.27 g (hydroxyl n°370/g) of AIONICO GL/609 (purchased from Lamberti SpA) and 0.145 g (0.762 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 3.70 g of a yellow oil (yield 90%).

¹H-NMR (DMSO-d₆, δ ppm): 3.10-3.78 (m, 11.55H), 4.00-4.56 (m, 2H), 6.70-7.54 (m, 15H), 7.54-8.00 (m, 3H).

### Example 7

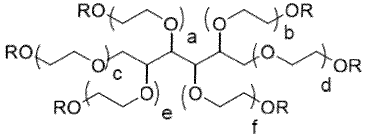

R =

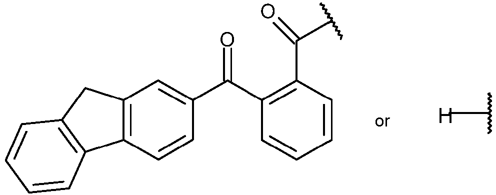

3.00 g (9.544 mmoles) of Intermediate 2, 2.14 g (hydroxyl n°300/g) of sorbilene RE/20 (purchased from Lamberti SpA) and 0.182 g (0.957 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.96 g of a brown-yellow oil (yield 100%).

¹H-NMR (DMSO-d₆, δ ppm): 3.00-3.75 (m, 14.47H), 3.78-4.30 (m, 4H), 7.26-7.81 (m, 7H), 7.81-8.06 (m, 4H).

### Example 8

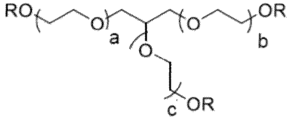

R =

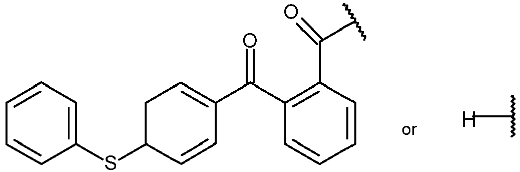

3.00 g (8.972 mmoles) of Intermediate 1, 1.50 g (hydroxyl n°370/g) of AIONICO GL/609 (purchased from Lamberti SpA) and 0.171 g (0.899 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.34 g of a pale oil (yield 100%).

¹H-NMR (DMSO-d₆, δ ppm): 3.05-3.75 (m, 11.6H), 4.00-4.30 (m, 2H), 7.10-7.83 (m, 12H), 7.83-8.03 (m, 1H).

### Example 9 (not according to the invention)

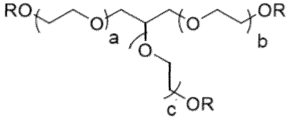

R =

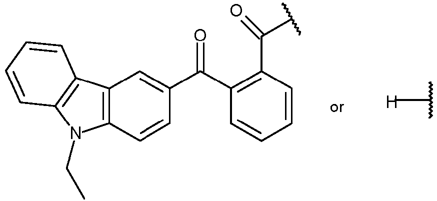

3.00 g (8.737 mmoles) of Intermediate 5, 1.52 g (hydroxyl n°370/g) of AIONICO GL/609 (purchased from Lamberti SpA) and 0.166 g (0.873 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.28 g of a yellow-orange oil (yield 98%).

¹H-NMR (DMSO-d₆, δ ppm): 1.25 (m, 3H), 2.90-3.79 (m, 10.15H), 3.90-4.62 (m, 4H), 7.04-7.29 (m, 1H), 7.30-7.82 (m, 7H), 7.82-8.58 (m, 3H).

### Example 10

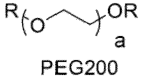

R =

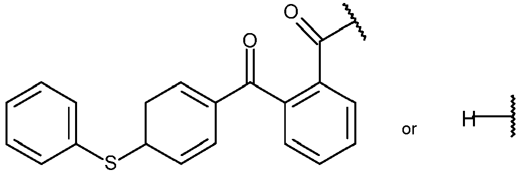

3.00 g (8.972 mmoles) of Intermediate 1, 0.942 g (4.710 mmoles) of Poly(ethylene glycol) (average mol wt 200) and 0.171 g (0.899 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 3.78 g of a colorless oil (yield 100%).

¹H-NMR (DMSO-d₆, δ ppm): 3.20-3.75 (m, 6.4H), 4.00-4.38 (m, 2H), 7.10-7.83 (m, 12H), 7.88-8.02 (m, 1H).

### Example 11

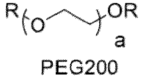

R =

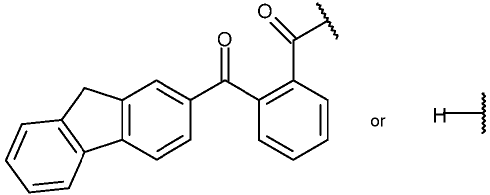

3.00 g (9.544 mmoles) of Intermediate 2, 1.000 g (5.000 mmoles) of Poly(ethylene glycol) (average mol wt 200) and 0.182 g (0.957 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 3.83 g of a pale oil (yield 100%).

¹H-NMR (DMSO-d₆, δ ppm): 3.15-3.75 (m, 4.4H), 3.80-4.38 (m, 4H), 7.30-7.53 (m, 3H), 7.53-7.82 (m, 4H), 7.87 (m, 1H), 7.90-8.07 (m, 3H).

### Example 12

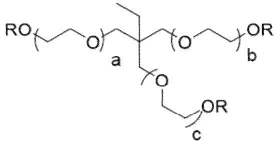

R =

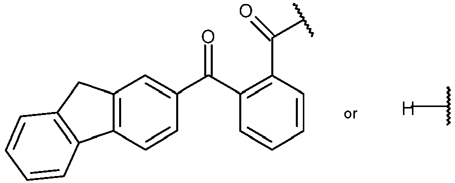

3.00 g (9.544 mmoles) of Intermediate 2, 1.691 g (hydroxyl n°364/g) of polyhol 3380 (purchased from PERSTORP) and 0.182 g (0.957 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.52 g of a pale oil (yield 100%).

¹H-NMR (DMSO-d₆, δ ppm): 0.44-0.91 (m, 3H), 1.00-1.45 (m, 2H), 2.88-3.75 (m, 33.5H), 3.76-4.38 (m, 9H), 7.30-7.44 (m, 4.5H), 7.44-7.81 (m, 11.25H), 7.81-8.09 (m, 9H).

### Example 13

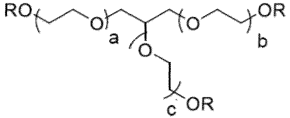

R =

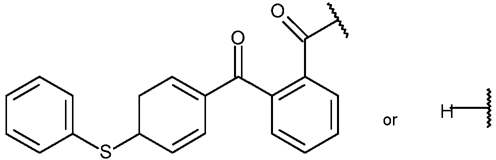

3.00 g (8.972 mmoles) of Intermediate 1, 1.645 g (3.290 mmoles) of Polyglycol G 500 (purchased from Clariant) and 0.171 g (0.899 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (15:15), 30 mL of sodium bicarbonate saturated solution and 30 mL of water. The organic layer was separated, dried over anhydrous sodium sulfate, filtered and the solvent removed by distillation under vacuum obtaining 4.48 g of a pale oil (yield 100%).

¹H-NMR (DMSO-d₆, δ ppm): 3.05-3.75 (m, 13H), 4.00-4.40 (m, 2H), 7.10-7.84 (m, 12H), 7.86-8.02 (m, 1H).

### Example 14

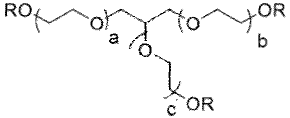

R =

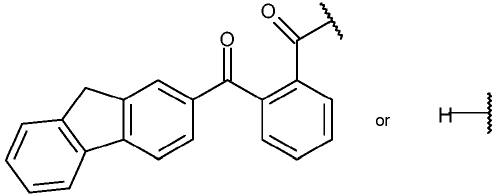

3.00 g (9.544 mmoles) of Intermediate 2, 1.829 g (3.658 mmoles) of Polyglycol G 500 (purchased from Clariant) and 0.182 g (0.957 mmoles) of p-toluenesulfonic acid monohydrate were mixed in 5 mL of toluene and refluxed until all water had been removed by azeotropic distillation. Then the reaction was allowed to cool, diluted with ethyl acetate and washed in sequence with 30 mL of a mixture of sodium bicarbonate saturated solution/water (1:1), 30 mL of sodium bicarbonate saturated solution and 30 mL of water. The organic layer was separated, dried over anhydrous sodium sulfate, filtered and the solvent removed by distillation under vacuum obtaining 4.66 g of a pale oil (yield 100%).

¹H-NMR (DMSO-d₆, δ ppm): 3.05-3.75 (m, 11.7H), 3.80-4.36 (m, 4H), 7.28-7.82 (m, 7H), 7.82-8.06 (m, 4H).

### Comparative Example 1

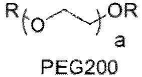

R =

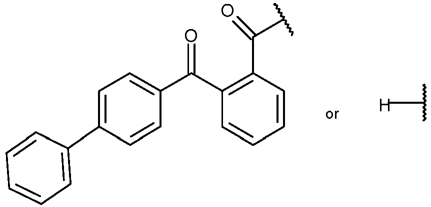

Comparative Example 1 was synthesized as disclosed in WO2015/010729.

### Test Example 1

### Yellow Index

The yellowing was measured as Yellow Index (YI) using a BYK color guide 45/0. The samples for the test were prepared as follows: in a formulation containing Photomer 6577 at 50% by wt, Photomer 4335, 15% by wt, Photomer 4666, 15% by wt, Photomer 4172 20% by wt the photoinitiator at 3 % by wt, the coinitiator (Esacure A198) at 3% by wt and Omnirad ITX (from IGM Resins B.V) at 0.5% By wt were added and stirred for 1 hours at 60°C. The formulation so obtained, was spread with a thickness of 12 microns on a varnished cardboard using a bar-coater and cured with a LED lamp (16 W/cm²) at the maximum speed at which the tack-free is reached. Then, the yellowing as Yellow Index (YI) is measured, the results are shown in Table 1.

**Table 1**

| UV LED Lamp 395 nm 16 W/cm² | Yellow index |
|---|---|
| Example 1 | 8.26 |
| Example 2 | 8.14 |
| Example 3 | 8.14 |
| Example 4 | 7.97 |
| Example7 | 8.12 |
| Example 8 | 8.03 |
| Example 12 | 8.59 |
| Example 10 | 8.53 |
| Example 13 | 8.59 |
| Comparative Example 1 | 10.01 |

The above test confirm that compounds of formula (I) surprising show a very lower yellowing after curing with respect to the comparative compound, being the Yellow Index in some case even 2 points lower, which is a particularly significant result.

### Test Example 2

### Through cure in cyan offset ink under Hg lamp

The test formulations were prepared dissolving the photoinitiator at a concentration of 3% by weight (wt) in an industrial cyan offset ink. The coinitiator (Esacure A198) was added at the compositions in the same amount (3% by weight). The test formulations were homogenized with a mechanical stirrer for 1 hour at 50°C and applied onto a white cardboard at 1.5 microns of thickness using IGT repro-tester equipment. The formulations were cured using a Mercury lamp (160 W/cm).

The through cure test is a measurement of the complete ink cure obtained at a defined speed and checked by "thumb twist pressure test". Higher speed corresponds to higher reactivity.

The results are shown in Table 2.

**Table2**

| Photoinitiator | Through-cure (m/min) |
|---|---|
| Example 11 | 80 |
| Comparative Example 1 | 55 |

This result also confirms the very good performance of the photoinitiators of the invention.

### Test example 3

### Surface curing in cyan offset ink under LED 365 nm lamp

The test formulations were prepared dissolving the photoinitiator at a concentration of 3% by wt in an industrial cyan offset ink. The coinitiator (Esacure A198) in the same amount (3% by wt) and Omnirad ITX at 0.5% by wt were added at the compositions. The test formulations were homogenized with a mechanical stirrer for 1 hour at 50°C and applied onto a white cardboard at 1.5 microns of thickness using IGT repro-tester equipment. The formulations were cured using a LED 365 nm lamp (12 W/cm2).

The evaluation was carried out considering the number of passages at a speed of 100 m/min to obtain a dry surface (cotton test). Lower is the number of passages, better the surface cure.

The results are shown in Table 3.

**Table 3**

| Photoinitiator | Surface cure, number of passages at 100 m/min |
|---|---|
| Example 1 | 3 |
| Comparative Example 1 | 5 |

This test also confirms that the compounds of formula (I) are more effective than the prior art Comparative compound.

## Claims

1. A compound of formula (I) 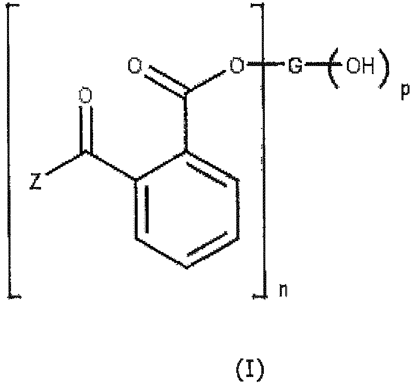 wherein
G is the residue of optionally ethoxylated and/or propoxylated monomeric, oligomeric or polymeric polyols G-(OH)ₙ₊ₚ;
n is from 1 to 8;
p is from 0 to 7;
n+p is from 2 to 8;
and Z is selected from (i), (ii) and (iii) 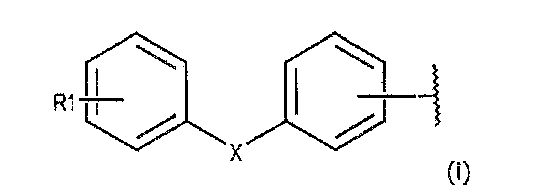 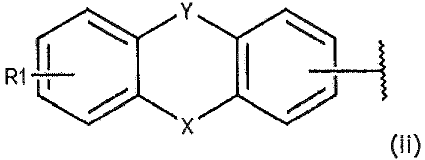 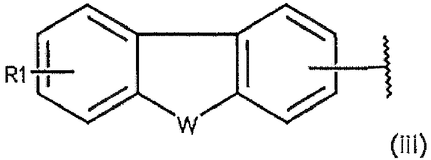
wherein
X, Y and W each independently, are selected from O, S and C(R2)(R3);
R1 is selected from hydrogen, halogen, substituted or unsubstituted C1-C20 alkyl, C5-C7 cycloalkyl, C3-C12 alkenyl, -O(C3-C12 alkenyl), -S(C3-C12 alkenyl), aryl, substituted or unsubstituted C1-C20 alkoxy, C1-C50 alkyl which is interrupted by one or more oxygens, C5-C7 cycloalkoxy, aryloxy, substituted or unsubstituted C1-C20 alkylthio, arylthio, substituted or unsubstituted (C1-C12alkyl)₂amino, (C5-C7cycloalkyl)₂amino, morpholino, piperidino, piperazino and N(C1-C12alkyl)piperazino;
R2 and R3 each independently, are selected from H, C1-C12 alkyl, OH and C1-C10 alkoxy;
and the wavy line shows the bonds linking to the keto groups of formula (I).

2. The compound of claim 1, **characterized in that** G-(OH)n+p is selected from a monomeric, an oligomeric, a polymeric polyol and mixtures thereof, which can be optionally ethoxylated and/or propoxylated, wherein
- n is from 1 to 6 and preferably from 2 to 6, more preferably 3 to 6, most preferably 3 to 5;
- p is from 0 to 6 and more preferably from 0 to 3; and
- n+p is from 2 to 8, and more preferably from 3 to 6.

3. The compound of any one of claims 1 to 3, **characterized in that** Z is (i) or (iii).

4. The compound of claim 1, **characterized in that** Z is (i) and X is S or O; or Z is (iii) and W is C(R2)(R3).

5. A photopolymerizable composition comprising:
a) from 50 to 99.9%, preferably from 70 to 98.9% by weight, based on the total content of the composition of at least one ethylenically unsaturated compound;
b) from 0.1 to 35%, preferably from 0.1 to 20%, and more preferably from 0.2 to 15% by weight, based on the total content of the composition, of at least one compound of formula (I) or a salt or solvate thereof, as defined in anyone of claims 1 to 4; and
c) from 0 to 20% by weight, preferably from 0 to 15%, and more preferably from 0.2 to 15% by weight, based on the total content of the composition, of an accelerator and/or of a coinitiator.

6. The photopolymerizable composition of claim 5, further comprising one or more of the following components:
d) from 0.01 to 15% by weight based on the total content of the composition, of one or more photosensitizer; and/or
e) from 0.5 to 15% by weight based on the total content of the composition, one or more further photoinitiators.

7. A process for photocuring photopolymerizable compositions, coatings, adhesives and inks, which process comprises:
(i) providing a photopolymerizable composition as defined in any one of claims 5 or 6;
(ii) coating or printing said photopolymerizable composition onto a substrate; and
(iii) photopolymerizing said coated or printed composition on said substrate with a light source.

8. A process for three-dimensional printing which comprises providing a photopolymerizable composition as defined in any one of claims 5 or 6 and photocuring said composition with a light source.

9. The process of claims 7 or 8, **characterized in that** said light source comprises UV light in at least one of the UVA, UVB and UVC ranges.

10. The process of any one of claims 7 to 10, **characterized in that** said light source is a LED source emitting in the range from 350 to 420 nm.

11. The process of any one of claims 7, 9 or 10, further comprising the step of applying said photopolymerizable composition to a substrate prior to photopolymerizing it.

12. An article of manufacture obtained according to the process of any one of claims 7 to 11.

## Patentansprüche

1. Verbindung der Formel (I) 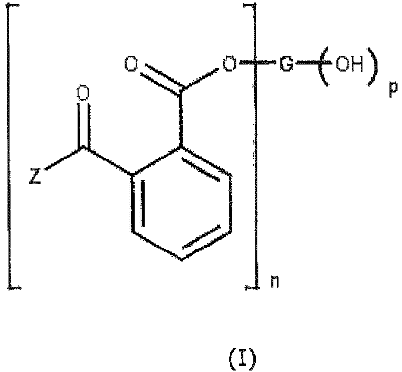 wobei
G der Rest von gegebenenfalls ethoxylierten und/oder propoxylierten monomeren, oligomeren oder polymeren Polyolen G-(OH)ₙ₊ₚ ist;
n von 1 bis 8 ist;
p von 0 bis 7 ist;
n+p von 2 bis 8 ist;
und Z ausgewählt ist aus (i), (ii) und (iii) 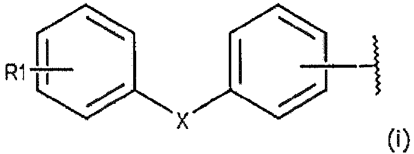 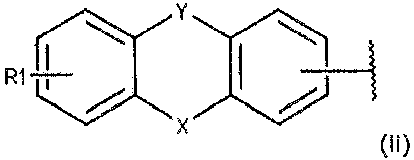 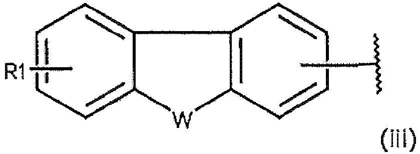
wobei
X, Y und W jeweils unabhängig voneinander ausgewählt sind aus O, S und C(R2)(R3);
R1 ausgewählt ist aus Wasserstoff, Halogen, substituiertem oder unsubstituiertem C1-C20-Alkyl, C5-C7-Cycloalkyl, C3-C12-Alkenyl, -O(C3-C12-Alkenyl), -S(C3-C12-Alkenyl), Aryl, substituiertem oder unsubstituiertem C1-C20-Alkoxy, C1-C50-Alkyl, das durch ein oder mehrere Sauerstoffatome unterbrochen ist, C5-C7-Cycloalkoxy, Aryloxy, substituiertem oder unsubstituiertem C1-C20-Alkylthio, Arylthio, substituiertem oder unsubstituiertem (C1-C12-Alkyl)₂amino, (C5-C7-Cycloalkyl)₂amino, Morpholino, Piperidino, Piperazino und N(C1-C12-Alkyl)piperazino;
R2 und R3 jeweils unabhängig voneinander ausgewählt sind aus H, C1-C12-Alkyl, OH und C1-C10-Alkoxy;
und die Wellenlinie die Bindungen zeigt, die mit den Ketogruppen der Formel (I) verbinden.

2. Verbindung nach Anspruch 1, **dadurch gekennzeichnet, dass** G-(OH)n+p ausgewählt ist aus einem monomeren, einem oligomeren, einem polymeren Polyol und Mischungen davon, die gegebenenfalls ethoxyliert und/oder propoxyliert sein können, worin
- n von 1 bis 6 und vorzugsweise von 2 bis 6, mehr bevorzugt 3 bis 6, am meisten bevorzugt 3 bis 5 ist;
- p von 0 bis 6 und mehr bevorzugt von 0 bis 3 ist; und
- n+p von 2 bis 8 und mehr bevorzugt von 3 bis 6 ist.

3. Verbindung nach einem der Ansprüche 1 bis 3, **dadurch gekennzeichnet, dass** Z (i) oder (iii) ist.

4. Verbindung nach Anspruch 1, **dadurch gekennzeichnet, dass** Z (i) ist und X S oder O ist; oder Z (iii) ist und W C(R2)(R3) ist.

5. Photopolymerisierbare Zusammensetzung, umfassend:
a) von 50 bis 99,9 %, vorzugsweise von 70 bis 98,9 Gew.-%, bezogen auf den Gesamtgehalt der Zusammensetzung, mindestens einer ethylenisch ungesättigten Verbindung;
b) von 0,1 bis 35 %, vorzugsweise von 0,1 bis 20 % und mehr bevorzugt von 0,2 bis 15 Gew.-%, bezogen auf den Gesamtgehalt der Zusammensetzung, mindestens einer Verbindung der Formel (I) oder eines Salzes oder Solvats davon, wie in einem der Ansprüche 1 bis 4 definiert; und
c) von 0 bis 20 Gew.-%, vorzugsweise von 0 bis 15 % und mehr bevorzugt von 0,2 bis 15 Gew.-%, bezogen auf den Gesamtgehalt der Zusammensetzung, eines Beschleunigers und/oder eines Coinitiators.

6. Photopolymerisierbare Zusammensetzung nach Anspruch 5, ferner umfassend eine oder mehrere der folgenden Komponenten:
d) von 0,01 bis 15 Gew.-%, bezogen auf den Gesamtgehalt der Zusammensetzung, eines oder mehrerer Photosensibilisatoren; und/oder
e) von 0,5 bis 15 Gew.-%, bezogen auf den Gesamtgehalt der Zusammensetzung, eines oder mehrerer weiterer Photoinitiatoren.

7. Verfahren zum Photohärten von photopolymerisierbaren Zusammensetzungen, Beschichtungen, Klebstoffen und Druckfarben, wobei das Verfahren umfasst:
(i) Bereitstellen einer photopolymerisierbaren Zusammensetzung, wie in einem der Ansprüche 5 oder 6 definiert;
(ii) Beschichten oder Bedrucken eines Substrats mit der photopolymerisierbaren Zusammensetzung; und
(iii) Photopolymerisieren der beschichteten oder bedruckten Zusammensetzung auf dem Substrat mit einer Lichtquelle.

8. Verfahren zum dreidimensionalen Drucken, umfassend das Bereitstellen einer photopolymerisierbaren Zusammensetzung, wie in einem der Ansprüche 5 oder 6 definiert, und das Photohärten der Zusammensetzung mit einer Lichtquelle.

9. Verfahren nach Anspruch 7 oder 8, **dadurch gekennzeichnet, dass** die Lichtquelle UV-Licht in mindestens einem der Bereiche UVA, UVB und UVC umfasst.

10. Verfahren nach einem der Ansprüche 7 bis 10, **dadurch gekennzeichnet, dass** die Lichtquelle eine LED-Quelle ist, die im Bereich von 350 bis 420 nm emittiert.

11. Verfahren nach einem der Ansprüche 7, 9 oder 10, ferner umfassend den Schritt des Aufbringens der photopolymerisierbaren Zusammensetzung auf ein Substrat vor deren Photopolymerisierung.

12. Erzeugnis, erhalten nach dem Verfahren gemäß einem der Ansprüche 7 bis 11.

## Revendications

1. Composé de formule (I) 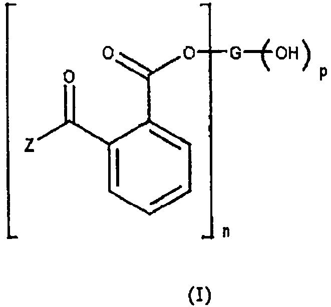 dans laquelle
G représente le résidu de polyols monomères, oligomères ou polymères, éventuellement éthoxylés et/ou propoxylés, de formule G-(OH)ₙ₊ₚ ;
N est compris entre 1 et 8 ;
P est compris entre 0 et 7 ;
n+p est compris entre 2 et 8 ;
et Z est choisi parmi (i), (ii) et (iii) 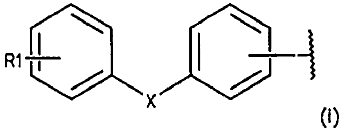 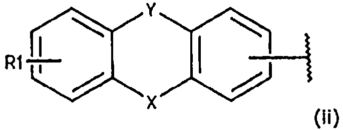 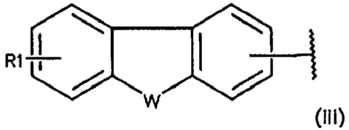
où
X, Y et W sont choisis chacun indépendamment parmi O, S et C(R2)(R3) ;
R1 est choisi parmi l'hydrogène, un halogène, un groupe alkyle en C1-C20 substitué ou non substitué en C1-C20, un groupe cycloalkyle en C5-C7, un groupe alcényle en C3-C12, -O(alcényle en C3-C12), -S(alcényle en C3-C12), un groupe aryle, un groupe alcoxy en C1-C20 substitué ou non substitué, un groupe alkyle en C1-C50 interrompu par un ou plusieurs atomes d'oxygène, un groupe cycloalcoxy en C5-C7, un groupe aryloxy, un groupe alkylthio en C1-C20 substitué ou non substitué, un groupe arylthio, un groupe (alkyle en C1-C12)₂amino substitué ou non substitué, un groupe (cycloalkyle en C5-C7)₂amino, morpholino, pipéridino, pipérazino et N(alkyle en C1-C12)pipérazino ;
R2 et R3 sont choisis chacun indépendamment parmi H, un groupe alkyle en C1-C12, OH et un groupe alcoxy en C1-C10 ;
et la ligne ondulée représente les liaisons reliant les groupes cétoniques de formule (I).

2. Composé selon la revendication 1, **caractérisé en ce que** G-(OH)n+p est choisi parmi un polyol monomère, oligomère ou polymère et des mélanges de ceux-ci, qui peuvent être éventuellement éthoxylés et/ou propoxylés, où
- n est compris entre 1 et 6, de préférence entre 2 et 6, plus préférablement entre 3 et 6, et de manière encore plus préférable entre 3 et 5 ;
- p est compris entre 0 et 6, et de préférence entre 0 et 3 ; et
- n+p est compris entre 2 et 8, et de préférence entre 3 et 6.

3. Composé selon l'une quelconque des revendications 1 à 3, **caractérisé en ce que** Z est (i) ou (iii).

4. Composé selon la revendication 1, **caractérisé en ce que** Z est (i) et X est S ou O ; ou Z est (iii) et W est C(R2)(R3).

5. Composition photopolymérisable comprenant :
a) de 50 à 99,9 %, de préférence de 70 à 98,9 % en poids, par rapport à la teneur totale de la composition, d'au moins un composé éthyléniquement insaturé ;
b) de 0,1 à 35 %, de préférence de 0,1 à 20 %, et plus préférablement de 0,2 à 15 % en poids, par rapport à la teneur totale de la composition, d'au moins un composé de formule (I) ou d'un sel ou solvate de celui-ci, tel que défini dans l'une quelconque des revendications 1 à 4 ; et
c) de 0 à 20 % en poids, de préférence de 0 à 15 %, et plus préférablement de 0,2 à 15 % en poids, par rapport à la teneur totale de la composition, d'un accélérateur et/ou d'un co-initiateur.

6. Composition photopolymérisable selon la revendication 5, comprenant en outre un ou plusieurs des composants suivants :
d) de 0,01 à 15 % en poids, par rapport à la teneur totale de la composition, d'un ou plusieurs photosensibilisateurs ; et/ou
e) de 0,5 à 15 % en poids, par rapport à la teneur totale de la composition, d'un ou plusieurs photo-initiateurs supplémentaires.

7. Procédé de photodurcissement de compositions, revêtements, adhésifs et encres photopolymérisables, lequel procédé comprend les étapes consistant à :
i) fournir une composition photopolymérisable telle que définie dans l'une quelconque des revendications 5 ou 6 ;
ii) appliquer ou imprimer ladite composition photopolymérisable sur un substrat ; et
iii) photopolymériser ladite composition appliquée ou imprimée sur ledit substrat à l'aide d'une source lumineuse.

8. Procédé d'impression tridimensionnelle qui comprend la fourniture d'une composition photopolymérisable telle que définie dans l'une quelconque des revendications 5 ou 6 et le photodurcissement de ladite composition à l'aide d'une source lumineuse.

9. Procédé selon l'une des revendications 7 ou 8, **caractérisé en ce que** ladite source lumineuse comprend de la lumière UV dans au moins l'une des gammes UVA, UVB et UVC.

10. Procédé selon l'une quelconque des revendications 7 à 10, **caractérisé en ce que** ladite source lumineuse est une source LED émettant dans la gamme de 350 à 420 nm.

11. Procédé selon l'une quelconque des revendications 7, 9 ou 10, comprenant en outre l'étape consistant à appliquer ladite composition photopolymérisable sur un substrat avant de la photopolymériser.

12. Article manufacturé obtenu selon le procédé selon l'une quelconque des revendications 7 à 11.
